# EUROPÄISCHE PATENTANMELDUNG

(11) **EP 4 477 639 A1**
(43) Veröffentlichungstag der Anmeldung: **18.12.2024**
(21) Anmeldenummer: 23179201.1
(22) Anmeldetag: 14.06.2023
(51) Int. Cl.: C07C 5/25, C07C 7/04, C07C 11/02

(54) **VERFAHREN ZUR BEREITSTELLUNG EINES STROMS, DER EINEN HOHEN ANTEIL AN 2,4,4-TRIMETHYLPENT-1-EN AUFWEIST**

(71) Anmelder: Evonik Oxeno GmbH & Co. KG, 45772 Marl (DE)
(72) Erfinder: KUCMIERCZYK, Peter, 44628 Herne (DE); FRANKE, Robert, 45772 Marl (DE); SALE, Anna Chiara, 45657 Recklinghausen (DE); FRIDAG, Dirk, 45721 Haltern am See (DE); MARKOVIC, Ana, 45721 Haltern am See (DE); RIX, Armin Matthias, 45770 Marl (DE); TERMÜHLEN, Maren, 44135 Dortmund (DE); HARDING, Laura-Selin, 46286 Dorsten (DE); METTERNICH, Jan Benedikt, 45657 Recklinghausen (DE)
(74) Vertreter: Evonik Patent Association

(57) **Zusammenfassung**

Gegenstand der Erfindung ist ein Verfahren zur Bereitstellung eines Kopfstroms K, der mehr als 85 Gew.-% an 2,4,4-Trimethylpent-1-en enthält, wobei sowohl eine Isomerisierung von 2,4,4-Trimethylpent-2-en zu 2,4,4-Trimethylpent-1-en als auch eine Destillation unter Erhalt des Kopfstroms K durchgeführt werden. Die Isomerisierung wird dabei zumindest teilweise mit dem Sumpfstrom der Destillation, bei der ein Kopfstrom K am Kopf abgenommen wird, gespeist.

## Beschreibung

Die vorliegende Erfindung betrifft ein Verfahren zur Bereitstellung eines Kopfstroms K, der mehr als 85 Gew.-% an 2,4,4-Trimethylpent-1-en enthält, wobei sowohl eine Isomerisierung von 2,4,4-Trimethylpent-2-en zu 2,4,4-Trimethylpent-1-en als auch eine Destillation unter Erhalt des Kopfstroms K durchgeführt werden. Die Isomerisierung wird dabei zumindest teilweise mit dem Sumpfstrom der Destillation, bei der ein Kopfstrom K am Kopf abgenommen wird, gespeist.

Di-isobuten ist ein technisch relevantes Erzeugnis, welches durch Dimerisierung von Isobuten gewonnen wird. Di-isobuten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 von etwa 78:22 bis 81 : 19 (Gleichgewichtsverteilung). Diese Mischung kann unter anderem in Carbonylierungsverfahren zu höherwertigen Produkten umgesetzt werden. Dabei weist speziell in Carbonylierungsverfahren, wie der Methoxycarbonylierung (Reaktionsprodukt ist hier der 3,5,5-Trimethylhesansäure-methylester) oder der Hydroformylierung (Reaktionsprodukt ist hier der 3,5,5-Trimethylhexanal) das innenständige Olefin TMP2 eine deutlich geringere Reaktivität als das endständige Olefin TMP1 auf. Oftmals können Reaktionsbedingungen oder Verweilzeiten bei diesen Carbonylierungsreaktionen aufgrund der mangelnden Stabilität der Katalysatoren oder aufgrund ökonomischer Faktoren (z. B. der Reaktorgröße) nicht dahingehend angepasst werden, dass das endständige TMP2 vollständig abreagiert.

Eine direkte Verwendung des unreagierten TMP2 ist aus Gründen der Reaktivität wenig sinnvoll. Daher empfiehlt es sich das TMP2 oder einen Teil davon in die reaktivere Form TMP1 mittels Isomerisierung zu überführen, um es schlussendlich zu werthaltigen Produkten umsetzen zu können.

Verfahren zur Isomerisierung von TMP2 zu TMP1 sind in der Literatur bekannt. Diese Verfahren werden jedoch homogen katalytisch und basierend auf starken mineralischen Säuren wie bspw. H₂SO4, H₃PO₄ oder Benzolsulfonsäure (vgl. US 2,554,251 A) durchgeführt. Homogenkatalytische Reaktionssysteme haben jedoch den Nachteil, dass die Isomerisierungskatalysatoren wieder aufwändig von dem erhaltenen reinem TMP1 oder den erhaltenen TMP1 /TMP2 Gemischen abgetrennt werden müssen. Erst dann könnten sie den bereits genannten Carbonylierungsverfahren zugeführt werden. Außerdem sind saure Systeme in der Regel korrosiv, sodass hochwertige Werkstoffe erforderlich sind

Ein weiteres Problem ist, dass der Anteil von TMP1 in dem Produktstrom der Isomerisierung in Abhängigkeit des nachgeschalteten Prozessschrittes, beispielsweise eines Carbonylierungsverfahrens, immer noch zu niedrig sein kann, um bei dem jeweiligen nachgeschalteten Prozessschritt vorteilhafte Ergebnisse erzielen zu können.

Die Aufgabe der vorliegenden Erfindung war deshalb die Bereitstellung eines Verfahrens zur Isomerisierung von 2,4,4-Trimethylpent-2-en zu 2,4,4-Trimethylpent-1-en, welches die vorgenannten Probleme nicht aufweist. Es sollte durch das Verfahren kontinuierlich ein Strom bereitgestellt werden, der einen hohen Anteil an 2,4,4-Trimethylpent-1-en aufweist. Gleichzeitig sollte das Verfahren die Möglichkeit bieten, weiteres 2,4,4-Trimethylpent-2-en zu 2,4,4-Trimethylpent-1-en umzusetzen.

Diese Aufgabe konnte durch das erfindungsgemäße Verfahren nach dem unabhängigen Anspruch 1 gelöst werden. Bevorzugte Ausführungsformen sind in den abhängigen Ansprüchen angegeben.

Erfindungsgemäß ist das Verfahren ein Verfahren zur Bereitstellung eines Kopfstroms K, der mehr als 85 Gew.-% an 2,4,4-Trimethylpent-1-en, vorzugsweise mehr als 90 Gew.-% an 2,4,4-Trimethylpent-1-en, besonders bevorzugt mehr als 95 Gew.-% an 2,4,4-Trimethylpent-1-en enthält, wobei das Verfahren zumindest die folgenden Schritte umfasst:
a. Zuführen eines Stroms A, welcher 2,4,4-Trimethylpent-2-en und 2,4,4-Trimethylpent-1-en enthält, zu einer Destillationseinheit und Auftrennen des Stroms A in mindestens den gegenüber dem Strom A an 2,4,4-Trimethylpent-1-en angereicherten Kopfstrom K, der mehr als 85 Gew.-% an 2,4,4-Trimethylpent-1-en, vorzugsweise mehr als 90 Gew.-% an 2,4,4-Trimethylpent-1-en, besonders bevorzugt mehr als 95 Gew.-% an 2,4,4-Trimethylpent-1-en enthält, und einen Strom B, der gegenüber dem Strom A an 2,4,4-Trimethylpent-1-en abgereichert ist;
b. Durchführung einer Isomerisierung von 2,4,4-Trimethylpent-2-en zu 2,4,4-Trimethylpent-1-en mit dem Strom B unter Verwendung eines heterogenen Katalysators auf Basis eines Zeoliths oder eines Ionenaustauscherharzes unter Erhalt eines Produktstroms der Isomerisierung, worin der Anteil des 2,4,4-Trimethylpent-2-ens geringer und der Anteil des 2,4,4-Trimethylpent-1-en höher ist als im Strom B; wobei
   der Produktstrom der Isomerisierung zumindest teilweise als Strom A zur Destillation geführt wird, und wobei
   ein Einsatzstrom, mit dem das Verfahren gespeist wird und der 2,4,4-Trimethylpent-2-en und 2,4,4-Trimethylpent-1-en enthält, zusätzlich zu Strom A zur Destillationseinheit in Schritt a. und/oder zusätzlich zu Strom B zur Isomerisierung in Schritt b. geführt wird.

Der Vorteil des erfindungsgemäßen Verfahrens ist die Verwendung eines heterogenen Katalysatorsystems. Ein solches Katalysatorsystem muss nicht vom Produktstrom abgetrennt werden, sondern verbleibt im Reaktionsgefäß bzw. dem Reaktor. Mit den erfindungsgemäßen Katalysatorsystemen auf Basis eines Zeoliths oder eines Ionenaustauscherharzes können zudem hohe Umsätze erzielt werden.

Ein weiterer Vorteil des Verfahrens ist die der Isomerisierung nachgeschaltete Destillation, wodurch der Anteil an 2,4,4-Trimethylpent-2-en und 2,4,4-Trimethylpent-1-en im Kopfstrom K der mindestens einen Destillationskolonne gegenüber dem eingesetzten Strom weiter gesteigert werden kann. So ist es möglich Anteile von mehr als 85 Gew-%, vorzugsweise mehr als 90 Gew.-%, besonders bevorzugt mehr als 95 Gew.-% an 2,4,4-Trimethylpent-1-en im Strom zu erreichen, was für nachgeschaltete Prozessschritte, z. B. Carbonylierungsverfahren, vorteilhaft ist.

Ein weiterer Vorteil ist die flexible Zuführung des Einsatzgemisches. Dadurch kann das erfindungsgemäße Verfahren an die jeweiligen Gegebenheiten angepasst werden, beispielweise in Abhängigkeit des eingesetzten Einsatzstroms. Liegen die Anteile von 2,4,4-Trimethylpent-2-en und 2,4,4-Trimethylpent-1-en im Einsatzstrom nah an der Gleichgewichtsverteilung, kann der Strom zunächst zur Destillation geführt werden. Bei von der Gleichgewichtsverteilung abweichenden Anteilen von 2,4,4-Trimethylpent-2-en und 2,4,4-Trimethylpent-1-en im Einsatzstrom könnte der Strom zunächst zur Isomerisierung geführt werden. Der Einbau in bestehende Produktionsanlagen ist somit in Abhängigkeit von der Zusammensetzung des Einsatzstroms einfacher möglich.

Schritt a. des erfindungsgemäßen Verfahrens betrifft die Destillation des Stroms A, welcher 2,4,4-Trimethylpent-2-en und 2,4,4-Trimethylpent-1-en enthält. Strom A wird dabei in mindestens den gegenüber dem Strom A an 2,4,4-Trimethylpent-1-en angereicherten Kopfstrom K, der mehr als 85 Gew.-% an 2,4,4-Trimethylpent-1-en, vorzugsweise mehr als 90 Gew.-% an 2,4,4-Trimethylpent-1-en, besonders bevorzugt mehr als 95 Gew.-% an 2,4,4-Trimethylpent-1-en enthält, und einen Strom B, der gegenüber dem Strom A an 2,4,4-Trimethylpent-1-en abgereichert ist, aufgetrennt.

Strom A, mit dem die Destillation gespeist wird, entstammt erfindungsgemäß der Isomerisierung. Der Strom, der in der Isomerisierung als Produktstrom erhalten wird, wird zumindest teilweise oder vollständig als Strom A zur Destillation geführt. Es ist erfindungsgemäß möglich, dass zusätzlich zu Strom A ein Einsatzstrom, mit dem das Verfahren gespeist wird und der 2,4,4-Trimethylpent-2-en und 2,4,4-Trimethylpent-1-en enthält, zur Destillationseinheit in Schritt a. geführt wird. Weiterhin ist es möglich, dass der Einsatzstrom zur Isomerisierung in Schritt b. geführt wird oder dass der Einsatzstrom sowohl zur Destillation in Schritt a. als auch zur Isomerisierung in Schritt b. geführt wird.

Der einzusetzende Einsatzstrom enthält 2,4,4-Trimethylpent-2-en und 2,4,4-Trimethylpent-1-en. Derartige Ströme können Diisobutenströme sein, welche mittels Dimerisierung aus Isobuten oder Isobutenhaltigen Kohlenwasserstoffgemischen hergestellt werden können, beispielsweise so wie es in EP 1 360 160 B1 offenbart wird. Weiterhin können die hier einzusetzenden Ströme als nicht reagierte Restströme bei Carbonylierungsverfahren anfallen, beispielsweise bei der Methoxycarbonylierung oder bei der Hydroformylierung.

Es ist bereits erwähnt worden, dass der Einsatzstrom zur Isomerisierung und/oder zur Destillation zugeführt werden kann. Die Bezeichnung der Schritte a. und b. im vorliegenden Anspruch stellt somit keine Priorisierung oder Chronologie dar. Es versteht sich, dass beide Schritte durchgeführt werden müssen und dass die einzelnen Schritte aus dem jeweils anderen Schritt gespeist werden. Welcher Schritt also als zuerst ausgeführt angesehen wird, ist daher nebensächlich.

Die Destillation in Schritt a. des erfindungsgemäßen Verfahrens wird in einer Destillationseinheit durchgeführt. Entsprechende Einheiten sind dem Fachmann grundsätzlich bekannt. Die Destillationseinheit der vorliegenden Erfindung umfasst vorzugsweise mindestens eine Destillationskolonne, besonders bevorzugt mindestens zwei Destillationskolonnen. Die nachfolgende Beschreibung von Merkmalen der Destillationskolonne gilt immer auch für den Fall, dass mehr als eine Destillationskolonne in der Destillationseinheit vorliegt.

Die Destillationskolonne weist vorzugsweise Einbauten auf, um die Trennaufgabe zu bewältigen. Entsprechende Einbauten sind dem Fachmann geläufig. Besonders geeignet sind hier regellose Packungen oder Strukturpackungen, wie sie dem Fachmann unter Handelsnamen wie MellaPak^{®}, MellapakPlus^{®}, Flexipac^{®} etc. bekannt sind. In einer bevorzugten Ausführungsform der vorliegenden Erfindung umfasst die mindestens eine Destillationskolonne mindestens 50 theoretische Trennstufen, vorzugsweise mindestens 70 theoretische Trennstufen, besonders bevorzugt mindestens 90 theoretische Trennstufen. Liegen zwei oder mehr Destillationskolonnen vor, können die Destillationskolonnen eine identische oder eine unterschiedliche Anzahl an theoretischen Trennstufen aufweisen.

Die Betriebsparameter der Destillationskolonne orientieren sich an der Trennaufgabe und der Gestaltung der Destillationskolonne. Im vorliegenden Verfahren ist es bevorzugt, dass die mindestens eine Destillationskolonne bei Unterdruck, besonders bevorzugt bei einem Druck von 0,2 bis 0,9 bar betrieben wird. Unterdruck liegt im Rahmen der vorliegenden Erfindung immer dann vor, wenn unterhalb des Umgebungsdrucks von ca. 1 bar, also dem an dem jeweiligen Standort vorliegenden Atmosphärendruck gearbeitet wird. Liegen zwei oder mehr Destillationskolonnen vor, können die Destillationskolonnen beim gleichen oder bei einem unterschiedlichen Druck betrieben werden.

Es ist weiterhin bevorzugt, dass die Temperatur im Sumpf der mindestens einen Destillationskolonne im Bereich von 50 bis 100 °C liegt. Liegen zwei oder mehr Destillationskolonnen vor, können die Destillationskolonnen bei dergleichen oder bei unterschiedlicher Temperatur betrieben werden.

Ein weiterer Parameter bei der Auslegung von Destillationskolonnen ist das Rücklaufverhältnis. Das Rücklaufverhältnis meint das Verhältnis Rücklauf (Rezyklat) zum Destillat (abgezogenes Kondensat, hier also der Kopfstrom K). Der Rücklauf ist also ein auskondensierter Teil des Kopfstroms, der in die Destillationskolonne zurückgeführt wird. Es ist erfindungsgemäß bevorzugt, dass das Rücklaufverhältnis der mindestens einen Destillationskolonne bei der Destillation in Schritt a. im Bereich von 5 bis 15 beträgt.

Durch die erfindungsgemäße Destillation in Schritt a. fällt an der mindestens einen Destillationskolonne ein Kopfstrom K an, der mindestens 85 Gew.-% an 2,4,4-Trimethylpent-1-en, vorzugsweise mindestens 90 Gew.-%an 2,4,4-Trimethylpent-1-en, vorzugsweise mindestens 95 Gew.-% an 2,4,4-Trimethylpent-1-en enthält. Weiterhin fällt bei der Destillation ein Strom B an, der gegenüber dem Strom A an 2,4,4-Trimethylpent-1-en abgereichert ist.

Der zusätzlich bei der Destillation in Schritt a. anfallende Strom B kann als Sumpfstrom oder als Seitenstrom an der mindestens einen Destillationskolonne abgenommen werden. Sind zwei Destillationskolonnen vorhanden, wird Strom B, unabhängig davon, ob als Seitenstrom oder Sumpfstrom abgenommen, in der letzten Destillationskolonne anfallen. Wird Strom B als Seitenstrom abgenommen kann er grundsätzlich auf gleicher Höhe oder unterhalb des Einlasses für den Strom A angeordnet sein. Dabei versteht sich, dass Zulauf und Ablauf ausreichend beanstandet voneinander angeordnet sein müssen. Strom B, unabhängig davon, ob als Seitenstrom oder Sumpfstrom abgenommen, enthält vorzugsweise 80 bis 92 Gew.-% 2,4,4-Trimethylpent-2-en.

Wird Strom B bei der Destillation in Schritt a. als Sumpfstrom der mindestens einen Destillationskolonne abgenommen, wird vorzugsweise vor oder nach der Isomerisierung ein Purgestrom aus dem Strom B abgezogen, der während der Isomerisierung entstandene Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Wird Strom B bei der Destillation in Schritt a als Seitenstrom der mindestens einen Destillationskolonne abgenommen, wird vorzugsweise im Sumpf der mindestens einen Destillationskolonne ein Strom anfallen, der die während der Isomerisierung entstandenen Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Destillationskolonnen werden bekanntermaßen im Sumpf beheizt, um die Trennaufgabe bewältigen zu können. Möglich ist hier eine Energiezufuhr über Heizdampf, der oft an chemischen Produktionsstandorten verfügbar ist. Dazu wird ein Teil des Sumpfes über einen Wärmetauscher (Sumpfverdampfer) geführt, dort erhitzt und dann zurück in den Sumpf der Destillationskolonne geführt. Um den Energiebedarf und/oder um die COz-Emissionen zu verringern, kann eine Wärmeintegration bei der Destillation in Schritt a. vorgesehen werden. Wärmeintegration bedeutet, dass Energie, die innerhalb des Verfahrens entsteht oder vorhanden ist, an anderer Stelle eingesetzt wird. Im vorliegenden Fall eignet sich vor allem die (freiwerdende) Kondensationsenergie, die am Kopf mindestens einer Destillationskolonne der Destillationseinheit anfällt. In einer bevorzugten Ausführungsform der vorliegenden Erfindung wird in Schritt a. also eine Wärmeintegration dergestalt durchgeführt, dass zumindest ein Teil der Kondensationsenergie am Kopf zum Erhitzen des Sumpfs genutzt wird.

Eine andere Möglichkeit zur Wärmeintegration besteht in der sogenannten Brüdenverdichtung, bei der zumindest ein Teil des Brüdens (Kopfstroms) verdichtet, also auf ein höheres Druckniveau gebracht, und optional erhitzt wird. Der so verdichtete und optional erhitzte Brüden wird zum Wärmetauscher (Sumpfverdampfer) geführt, um damit den Sumpf zu erhitzen. Hierzu wird die Kondensationswärme des Brüden genutzt.

Eine weitere Möglichkeit besteht in dem Einsatz einer Wärmepumpe. Wärmepumpen werden mit einem Arbeitsmedium, z. B. n-Butan oder Wasser betrieben. Die Kondensationsenergie wird hier also zunächst in einem Wärmetauscher auf ein Arbeitsmedium übertragen und von diesem in einem weiteren geeigneten Wärmetauscher auf den Sumpf in der Destillation. Das Arbeitsmedium wird dabei in aller Regel über einen Verdichter zum Wärmetauscher im Sumpf geführt. Es ist grundsätzlich auch möglich zweistufige oder mehrstufige Wärmepumpen einzusetzen, bei denen mehr als eine Kompressorstufe vorliegt. Bei einer zweistufigen Wärmepumpe liegt nicht nur ein Arbeitsmedium, sondern zwei Arbeitsmedien vor, wobei zwischen dem ersten und dem zweiten Arbeitsmedium ebenfalls ein Energieaustausch in einem Wärmetauscher stattfindet. Bei mehrstufigen Ausgestaltungen sind entsprechend mehr Arbeitsmedien vorhanden.

Die Isomerisierung in Schritt b. wird mit dem Strom B durchgeführt, der gegenüber dem Strom A an 2,4,4-Trimethylpent-1-en abgereichert ist und aus der Destillation in Schritt a. entnommen wird. Zusätzlich zu Strom B kann der Einsatzstrom teilweise oder vollständig zur Isomerisierung geführt werden.

Die Isomerisierung kann weiterhin grundsätzlich in jedem geeigneten Reaktor durchgeführt werden. Möglich ist, dass die Isomerisierung in einem einzigen Reaktor oder in mehreren parallel oder in Reihe geschalteten Reaktoren erfolgt. Möglich ist zudem die Durchführung in Chargen oder im kontinuierlichen Betrieb. Bevorzugt wird die Isomerisierung in einem oder mehreren kontinuierlich arbeitenden Reaktoren, die üblicherweise bei Feststoff/Flüssigkeits-Kontaktreaktionen zum Einsatz gelangen, durchgeführt. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben. Ein anderes Konzept als Festbettreaktoren sind beispielsweise Reaktoren, in denen der Ionenaustauscher oder Zeolith suspendiert in einer flüssigen Phase vorliegt.

Als Reaktoren werden bei der erfindungsgemäßen Isomerisierung bevorzugt Rohrreaktoren oder Rohrbündelreaktoren, insbesondere solche mit inneren Rohrdurchmessern von 10 bis 60 mm, verwendet. Das Verhältnis von Länge zu Durchmesser der Katalysatorschüttung kann dabei variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht und der Wärmeübergang an das Kühlmedium gezielt beeinflusst werden.

Die Kühlung der Rohre des Reaktors, sei es Rohrreaktor oder Rohrbündelreaktor, kann über ein Kühlmedium (z.B. Kühlwasser oder zur Wärmeintegration ein wärmeaufnehmendes Prozessfluid) über den Mantelraum des Reaktors oder einen Wärmeübertrager in einem externen Recycle erfolgen. Insbesondere bei Einsatz von flüssigen Heizmedien wird dabei die Mantelseite konstruktiv so ausgeführt, dass ein möglichst homogener Temperaturgradient an allen Rohren anliegt. Die hierfür notwendigen technischen Maßnahmen sind dem Fachmann bekannt und in der Literatur beschrieben (Einbau von Umlenkblechen, Disc- an Donut-Bauweise, Einspeisung / Abfuhr des Wärmeträgers an verschiedenen Stellen des Reaktors usw.). Bevorzugt werden Reaktionsmedium und Wärmeträger im Gleichstrom, besonders bevorzugt von oben nach unten durch die Reaktorrohre bzw. den Reaktormantel geführt. Eine bevorzugte Ausführungsform ist beispielsweise in DE 10 2006 040 433 A1 beschrieben.

Die Isomerisierung von 2,4,4-Trimethylpent-2-en zu 2,4,4-Trimethylpent-1-en erfolgt exotherm, verläuft also unter Freisetzung von Energie, was zu einer Erwärmung der Reaktionsmischung führt. Zur Begrenzung des Temperaturanstiegs ist die Verdünnung des Einsatzstroms, beispielsweise durch Rückführung von Produkt möglich.

Der oder die bei der Isomerisierung eingesetzten Reaktor(en) können adiabatisch, polytrop oder praktisch isotherm betrieben werden. Praktisch isotherm bedeutet, dass die Temperatur an einer beliebigen Stelle im Reaktor maximal um 10°C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren eine Kühlung vorzusehen. Reaktoren, die für einen polytropen oder praktisch isothermen Betrieb geeignet sind, sind beispielsweise die bereits erwähnten Rohrbündelreaktoren, aber auch Rührkessel- und Schlaufenreaktoren.

Das Verfahren kann bei eher milden Temperaturen durchgeführt werden. Die Isomerisierung in Schritt b wird vorzugsweise bei einer Temperatur von 25 bis 90 °C, vorzugsweise 30 bis 80 °C, besonders bevorzugt 35 bis 70 °C durchgeführt.

Weiterhin kann die erfindungsgemäße Isomerisierung bei einem Druck gleich oder größer als der Dampfdruck des Einsatzstromgemisches und/oder des Reaktionsgemisches bei der jeweiligen Reaktionstemperatur durchgeführt werden, vorzugsweise bei einem Druck von mehr als 0 bar aber weniger als 40 bar.

Weiterhin bevorzugt wird die Isomerisierung in Schritt b. in der Flüssigphase durchgeführt. Es sollte klar sein, dass in diesem Fall Druck und Temperatur so zu wählen sind, dass der Einsatzstrom in der flüssigen Phase vorliegt bzw. vorliegen kann.

In dem oder den Reaktor(en) können bei der erfindungsgemäßen Isomerisierung auch verschiedene Katalysatoren auf Basis eines Zeoliths oder eines Ionenaustauscherharzes eingesetzt werden. So kann beispielsweise ein Gemisch von lonenaustauscherharzen unterschiedlicher Reaktivität eingesetzt werden. Ebenso ist es möglich, dass ein Reaktor Katalysatoren mit unterschiedlicher Aktivität enthält, die beispielsweise in Schichten angeordnet sind. Wird mehr als ein Reaktor verwendet, können die einzelnen Reaktoren mit dem gleichen oder unterschiedlichen Katalysator(en) auf Basis eines Zeoliths oder eines Ionenaustauscherharzes gefüllt sein.

Als heterogener Katalysator wird bei der erfindungsgemäßen Isomerisierung ein Katalysator auf Basis eines Zeoliths oder eines Ionenaustauscherharzes eingesetzt. Entsprechende Zeolithe und lonenaustauscherharze sind zahlreich im Handel verfügbar und dem Fachmann bekannt. Es hat sich gezeigt, dass der Katalysator auf Basis eines Zeoliths vorzugsweise ein Si : AI Verhältnis im Bereich von 40 : 1 bis 200 : 1 aufweist. Weiterhin hat sich gezeigt, dass als Katalysator auf Basis eines Ionenaustauscherharzes vorzugsweise der Styrol-Divinylbenzol-Typ als H-Form und in teilneutraliserter Form gut geeignet ist.

Ist der Katalysator ein Zeolith, haben sich einige Zeolithe als besonders vorteilhaft herausgestellt, beispielsweise beta- und gamma-Zeolithe. Der Zeolith wird daher vorzugsweise aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt.

Als lonenaustauscherharz können beispielsweise lonenaustauscherharze eingesetzt werden, solche, die durch die Sulfonierung von Phenol/Aldehyd-Kondensaten oder durch die Sulfonierung von Copolymeren von aromatischen Vinylverbindungen hergestellt werden. Beispiele für aromatische Vinylverbindungen zur Herstellung der Copolymere sind: Styrol, Vinyltoluol, Vinylnaphthalin, Vinylethylbenzol, Methylstyrol, Vinylchlorbenzol, Vinylxylol und Divinylbenzol. Besonders bevorzugt werden lonenaustauscherharze für die Isomerisierung eingesetzt, die durch die Sulfonierung von Copolymerenn, die durch Umsetzung von Styrol mit Divinylbenzol entstehen, hergestellt werden. Die lonenaustauscherharze können gelförmig, makroporös oder schwammförmig hergestellt werden. Die Eigenschaften dieser Harze, insbesondere spezifische Oberfläche, Porosität, Stabilität, Quellung oder Schrumpfung und Austauschkapazität, können bekanntermaßen durch den Herstellprozess variiert werden.

Ionenaustauscherharze des bevorzugten Styrol-Divinylbenzol-Typs werden u. a. unter folgenden Handelsnamen verkauft: CT 151 und CT275 der Firma Purolite, Amberlyst^{®} 15, Amberlyst^{®} 35, Amberlite^{®} IR-120, Amberlite^{®} 200 der Firma Rohm&Haas, Dowex M-31 der Firma DOW, LEWATIT^{®} K 2621, LEWATIT^{®} K 2431der Firma Lanxess.

Das Porenvolumen der als Katalysatoren einsetzbaren lonenaustauscherharze, insbesondere des bevorzugten Styrol-Divinylbenzol-Typs, beträgt vorzugsweise 0,3 bis 0,9 ml/g, besonders bevorzugt 0,5 bis 0,9 ml/g. Das Porenvolumen kann beispielsweise mittels adsorptiver Techniken ermittelt werden. Die Korngröße der lonenaustauscherharze beträgt bevorzugt van 0,3 mm bis 1,5 mm, besonders bevorzugt 0,5 mm bis 1,0 mm. Die Korngrößenverteilung kann enger oder weiter gewählt werden. So können beispielsweise lonenaustauscherharze mit sehr einheitlicher Korngröße (monodisperse Harze) eingesetzt werden.

Die als Katalysatoren bei der Isomerisierung einsetzbaren lonenaustauscherharze können als teilneutralisierte lonenaustauscherharze vorliegen. Dazu kann das lonenaustauscherharz mit Säuren oder Basen behandelt werden, wie es in der EP 1 360 160 B1 beschrieben wird.

Aus der Isomerisierung in Schritt b. wird dann ein Produktstrom erhalten, bei dem der Anteil des 2,4,4-Trimethylpent-2-ens geringer und der Anteil des 2,4,4-Trimethylpent-1-ens im Produktstrom höher ist als im eingesetzten Strom B. Der Produktstrom aus der Isomerisierung wird dann teilweise oder vollständig der Destillation in Schritt a. zugeführt.

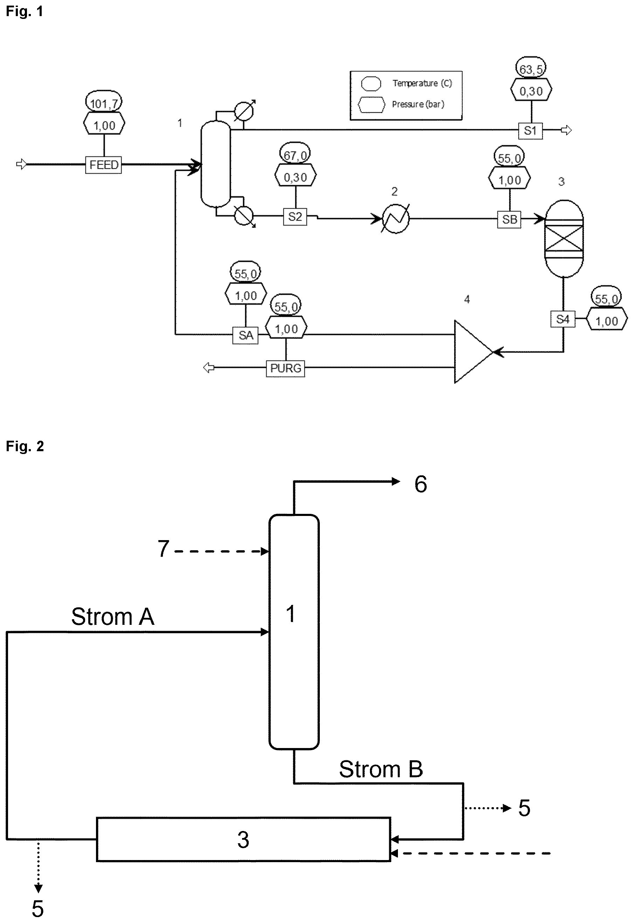

Die vorliegende Erfindung kann anhand der Abbildungen Fig. 2, Fig. 3 und Fig. 4 näher erläutert werden. Fig. 1 zeigt das Flussdiagramm, auf dessen Grundlage die Simulationen in Beispiel 2 erstellt worden sind und wird bei der Versuchsbeschreibung erklärt.

Fig. 2 zeigt die Ausführungsform, bei der der Strom B als Sumpfstrom aus der Destillation (1) entnommen wird. Strom B wird dann zur Isomerisierung (3) geführt. Der dort erhaltene Produktstrom wird zur Destillation (1) geleitet. Bei der Bildung von Hochsiedern kann vor oder nach der Isomerisierung (3) ein Purgestrom (5) entnommen werden. Dies wird durch die gestrichelten Linien angedeutet. Vorteilhafter ist die Abtrennung der Hochsieder vor der Isomerisierung. Destillation (1) und/oder Isomerisierung (3) werden zudem mit dem Einsatzstrom (7) gespeist. Aus der Destillation wird dann der gewünschte Kopfstrom K (6) entnommen.

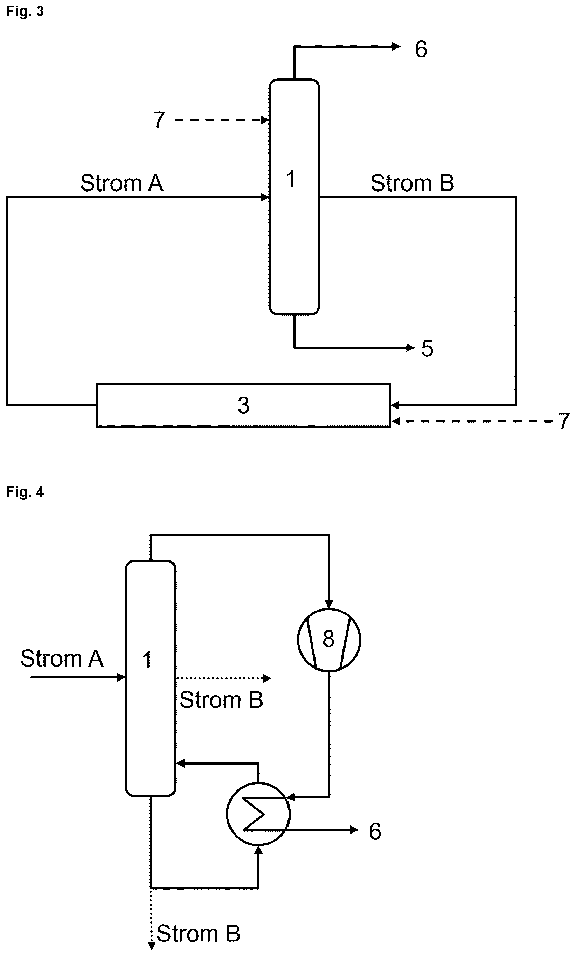

Fig. 3 zeigt die Ausführungsform, bei der der Strom B als Seitenstrom aus der Destillation (1) entnommen wird. In dieser Ausführungsform werden eventuell anfallende Hochsieder als Purge (5) im Sumpf der Destillation (1) anfallen. Alles weitere ist identisch zur Ausführungsform nach Fig. 2.

Fig. 4 zeigt einen Ausschnitt der in Fig. 2 und 3 dargestellten Ausführungsformen, wobei im Unterschied zu diesen Ausführungsformen hier eine Brüdenverdichtung vorliegt. Der Brüden wird dabei über den Verdichter (8) zum Sumpf geführt und Energie im Wärmetauscher auf den Sumpf übertragen. Der Brüden (6) wird kondensiert und als TMP-1-reiche Fraktion aus dem Verfahren ausgeschleust. Bei Strom B sind die in Fig. 2 und 3 beschriebenen Möglichkeiten für die Abnahme des Stroms aus der Destillation durch gestrichelte Linien angedeutet.

Die Erfindung wird nachfolgend anhand von Beispielen beschrieben. Diese dienen der Erläuterung und stellen keine Einschränkung des Erfindungsgegenstands dar.

### Beispiele

### Beispiel 1

2,4 g eines erfindungsgemäßen Katalysators (Purolite CT 275, teilneutralisiert, 0.9 eq/L) wurden in einem Rohrreaktor mit einem Durchmesser von 0,6 cm und einer Höhe von 12 cm eingefüllt. Hieraus ergibt sich ein Leerrohrvolumen von 3,39 cm³. Der Reaktor wurde mit 2,4,4-Trimethylpten-2-en (>99%) mit einem Förderstrom von 0,1 g/min beschickt. Daraus ergibt sich eine Leerrohrverweilzeit von 24,5 min. Die Isomerisierung erfolgte bei einer Temperatur von 55 °C und Umgebungsdruck. Nach Austritt aus dem Reaktor wurde die Verteilung von 2,4,4-Trimethylpent-2-en zu 2,4,4-Trimethylpent-1-en bestimmt (im Eingangsstrom 2,4,4-Trimethylpent-2-en : 2,4,4-Trimethylpent-1-en 99 : 1), was in Tabelle 1 gezeigt ist. Die Analyse erfolgte durch Gaschromatographie. Ausgewertet wurden die Peak-Flächen nach Methode der externen Kalibration.

**Tabelle 1: Anteil der Isomere im Di-isobuten**

| Massen-Strom 2,4,4-Trimethylpent-2-en [g/min] | Temperatur [°C] | w-Anteil 2,4,4-Trimethylpent-2-en an Di-isobuten-Isomeren im Produktstrom [%] | w-Anteil 2,4,4-Trimethylpent-1-en an Di-isobuten-Isomeren im Produktstrom [%] |
|---|---|---|---|
| 0,1 | 55 | 22^{a} | 78^{a} |

| | | | |
|---|---|---|---|
| ^{a}Über 80 Stunden Versuchszeit. | | | |

Die Ergebnisse zeigen, dass der erfindungsgemäße Katalysator sehr gut für die Isomerisierung geeignet ist.

### Beispiel 2

Zur Simulation wurden die Stoffdaten, die innerhalb der Evonik zur Verfügung stehen verwendet. Als Simulationstool wurde Aspen Plus in der Version V10 benutzt. Der Isomerisierungsreaktor wurde entsprechend den experimentellen Daten aus Beispiel 1 (Purolite CT 275, teilneutralisiert, 0,9 eq/L) spezifiziert. Entsprechend Fig. 1 ist ein Prozess zu sehen, in dem Di-isobuten zunächst in 2,4,4-Trimethylpent-1-en und 2,4,4-Trimethylpent-2-en destillativ aufgetrennt wird, um den am Sumpf mit TMP2 angereicherten Strom über einen heterogenen Katalysator in einen wieder an TMP1 angereicherten Strom zu überführen und diesen dann erneut der destillativen Trennung zuzuführen, woraus sich ein an TMP1 angereicherter Produktstrom ergibt.

Der Einsatzstrom "FEED" besteht aus einem Gemisch aus TMP1 und TMP2 in einem Verhältnis von 81 Gew.-% TMP1 und 19 Gew.-% TMP2. Dieser wird der Destillationskolonne (1), die mit 100 theoretischen Trennstufen bei einem Rücklaufverhältnis von 8 betrieben wird, über Stufe 50 zugeführt. Die Destillationskolonne (1) wird bei 0,3 bar sowie bei einer Sumpftemperatur von 67 °C und einer Kopftemperatur von 65,3 °C betrieben. Am Sumpf wird der Strom S2 mit 67 °C gewonnen mit einer Zusammensetzung von 9,13 Gew.-% TMP1 und 90,87 Gew.-% TMP2, über den Wärmetauscher (2) auf 55 °C abgekühlt und als Strom B dem Isomerisierungsreaktor (3) zugeführt. Der Isomerisierungsreaktor (3) ist mit einem heterogenen Katalysator (wie in Bsp. 1 genannt) gefüllt. Bei einer Leerrohrverweilzeit von > 24 min. ergibt sich Strom S4 mit einer Zusammensetzung von 78 Gew.-% TMP1 und 22 Gew.-% TMP2. Dieser Strom kann, falls ein Purge notwendig ist, über einen Splitter (4) (Faktor 0,03) in den Strom PURGE und den Strom A aufgeteilt werden. Strom A wird der Destillationskolonne (1) über der Stufe 40 wieder zugeführt. Am Kopf der Destillationskolonne (1) wird der vollkondensierte Kopfstrom K bei einer Temperatur von 63,5 °C gewonnen, wobei der Strom eine Zusammensetzung von 96 Gew.-% TMP1 und 4 Gew.-% TMP2 aufweist. Eine Übersicht über die Zusammensetzung der Ströme findet sich zudem in Tabelle 2.

**Tabelle 2: Übersicht über die einzelnen Ströme bei der Simulation in Beispiel 2**

| | | **FEED** | **Strom A (SA)** | **Kopfstrom K** | **S2** | **Strom B (SB)** | **S4** |
|---|---|---|---|---|---|---|---|
| | Einheiten | | | | | | |
| Phase | | Flüssig | Flüssig | Flüssig | Flüssig | Flüssig | Flüssig |
| Temperatur | °C | 101,7 | 55 | 63,5 | 67 | 55 | 55 |
| Druck | bar | 1 | 1 | 0.3 | 0.3 | 1 | 1 |

| Massenanteil | | | | | | | |
|---|---|---|---|---|---|---|---|
| TMP1 | | 0,81 | 0,78 | 0,96 | 0,09 | 0,09 | 0,78 |
| TMP2 | | 0,19 | 0,22 | 0,04 | 0,91 | 0,91 | 0,22 |

Es ist zu erkennen, dass mit dem erfindungsgemäßen Verfahren ein Kopfstrom K mit sehr hohem Gehalt an 2,4,4-Trimethylpent-1-en erhalten werden kann.

## Patentansprüche

1. Verfahren zur Bereitstellung eines Kopfstroms K, der mehr als 85 Gew.-% an 2,4,4-Trimethylpent-1-en enthält, wobei das Verfahren zumindest die folgenden Schritte umfasst:
a. Zuführen eines Stroms A, welcher 2,4,4-Trimethylpent-2-en und 2,4,4-Trimethylpent-1-en enthält, zu einer Destillationseinheit und Auftrennen des Stroms A in mindestens den gegenüber dem Strom A an 2,4,4-Trimethylpent-1-en angereicherten Kopfstrom K, der mehr als 85 Gew.-% an 2,4,4-Trimethylpent-1-en enthält, und einen Strom B, der gegenüber dem Strom A an 2,4,4-Trimethylpent-1-en abgereichert ist;
b. Durchführung einer Isomerisierung von 2,4,4-Trimethylpent-2-en zu 2,4,4-Trimethylpent-1-en mit dem Strom B unter Verwendung eines heterogenen Katalysators auf Basis eines Zeoliths oder eines Ionenaustauscherharzes unter Erhalt eines Produktstroms der Isomerisierung, worin der Anteil des 2,4,4-Trimethylpent-2-ens geringer und der Anteil des 2,4,4-Trimethylpent-1- höher ist als im Strom B; wobei
der Produktstrom der Isomerisierung zumindest teilweise als Strom A zur Destillation geführt wird, und wobei
ein Einsatzstrom, mit dem das Verfahren gespeist wird und der 2,4,4-Trimethylpent-2-en und 2,4,4-Trimethylpent-1-en enthält, zusätzlich zu Strom A zur Destillationseinheit in Schritt a. und/oder zusätzlich zu Strom B zur Isomerisierung in Schritt b. geführt wird.

2. Verfahren nach Anspruch 1, wobei die Destillationseinheit mindestens eine Destillationskolonne, vorzugsweise mindestens zwei Destillationskolonnen umfasst.

3. Verfahren nach Anspruch 1 oder 2, wobei die mindestens eine Destillationskolonne bei Unterdruck, vorzugsweise bei einem Druck von 0,2 bis 0,9 bar betrieben wird.

4. Verfahren nach einem der vorhergehenden Ansprüche, wobei die Temperatur im Sumpf der mindestens einen Destillationskolonne im Bereich von 50 bis 100 °C liegt.

5. Verfahren nach einem der vorhergehenden Ansprüche, wobei das Rücklaufverhältnis bei der mindestens einen Destillationskolonne 5 bis 15 beträgt.

6. Verfahren nach einem der vorhergehenden Ansprüche, wobei Strom B der mindestens einen Destillationskolonne 80 bis 92 Gew.-% 2,4,4-Trimethylpent-2-en enthält.

7. Verfahren nach einem der vorhergehenden Ansprüche, wobei Strom B als Sumpfstrom oder als Seitenstrom an der mindestens einen Destillationskolonne abgenommen wird.

8. Verfahren nach Anspruch 5, wobei Strom B als Sumpfstrom der mindestens einen Destillationskolonne abgenommen wird und vor oder nach der Isomerisierung ein Purgestrom abgezogen wird, der während des Isomerisierung entstandene Hochsieder enthält.

9. Verfahren nach Anspruch 5, wobei Strom B als Seitenstrom der mindestens einen Destillationskolonne abgenommen wird und im Sumpf der mindestens einen Destillationskolonne ein Strom anfällt, der der während des Isomerisierung entstandene Hochsieder enthält.

10. Verfahren nach einem der vorhergehenden Ansprüche, wobei die Isomerisierung in Schritt b bei einer Temperatur von 25 bis 90 °C, vorzugsweise 30 bis 80 °C, besonders bevorzugt 35 bis 70 °C durchgeführt wird.

11. Verfahren nach einem der vorhergehenden Ansprüche, wobei das Zeolith ein Si : AI Verhältnis im Bereich von 40 : 1 bis 200 : 1 aufweist.

12. Verfahren nach einem der vorhergehenden Ansprüche, wobei das Zeolith aus der Gruppe der beta- und gamma-Zeolithe ausgewählt wird.

13. Verfahren nach einem der vorhergehenden Ansprüche, wobei der Katalysator ein Zeolith ist und aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt wird.

14. Verfahren nach einem der vorhergehenden Ansprüche, wobei als lonenaustauscherharz solche eingesetzt werden, die durch die Sulfonierung von Phenol/Aldehyd-Kondensaten oder durch die Sulfonierung von Copolymeren von aromatischen Vinylverbindungen hergestellt werden.

15. Verfahren nach einem der vorhergehenden Ansprüche, wobei in Schritt a. eine Wärmeintegration dergestalt durchgeführt wird, dass zumindest ein Teil der Kondensationsenergie am Kopf zum Erhitzen des Sumpfs genutzt wird, vorzugsweise in Form einer Brüdenverdichtung oder durch Einsatz einer Wärmepumpe.
